(19) Europäisches Patentamt
European Patent Office
Office européen des brevets

(11) Publication number: **0 226 619 B1**

(12) **EUROPEAN PATENT SPECIFICATION**

(45) Date of publication of patent specification: **16.12.92** (51) Int. Cl.5: **C08G 18/32**

(21) Application number: **86903929.7**

(22) Date of filing: **27.05.86**

(86) International application number:
**PCT/US86/01175**

(87) International publication number:
**WO 86/07069 (04.12.86 86/26)**

(54) **Use of urethane compositions for bumper repair.**

(30) Priority: **31.05.85 US 739827**
**03.06.85 US 740256**

(43) Date of publication of application:
**01.07.87 Bulletin 87/27**

(45) Publication of the grant of the patent:
**16.12.92 Bulletin 92/51**

(84) Designated Contracting States:
**DE FR GB**

(56) References cited:
**GB-A- 2 147 596**
**US-A- 3 691 117**
**US-A- 4 225 696**
**US-A- 4 476 292**
**US-A- 31 389**
**US-A- 4 045 527**
**US-A- 4 400 498**

(73) Proprietor: **JACOBS, Richard**
**8894 regent Street**
**Los Angeles, CA 90034(US)**

(72) Inventor: **JACOBS, Richard**
**8894 regent Street**
**Los Angeles, CA 90034(US)**

(74) Representative: **Grussdorf, Jürgen, Dr. et al**
**Patentanwälte Zellentin & Partner Rubens-strasse 30**
**W-6700 Ludwigshafen(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention has to do with compositions useful for the repair of automobile bumpers and other body parts, and more particularly is concerned with special urethane polymer systems which afford rapid cure to sandable state, excellent adhesion and toughness in the auto body repair art.

It is known to use urethane polymers in the repair of bumpers and like synthetic organic polymer body components, although such use is far less than use of epoxy and other curable resins than urethane. Known urethanes, however, are not as hard as desired. The chemistry of the urethane polymer is such that attempting to make a harder polymer requires relatively higher amounts of isocyanate reagent. In the auto repair situation, however, relatively higher amounts of isocyanate are desirably avoided since cure times to a sandable stage are increased, necessitating delays in finishing the repair, the painting and so on and increasing labor costs. Thus presently used urethanes have been limited in hardness so as to get fast sandability. An important factor in determining utility of polymer compositions for auto body repair is the rapidity with which the composition reaches a sandable condition and the ability of the polymer composition to sand in extremely thin sections, near the edge of a patch for example where the material is nearly feathered. Other properties such as hardness, shelf life, broad tolerance for mixing variations, adhesion to the substrate, colorability, ease of application and clean up and reproducibility from batch to batch, together with convenient packaging for efficient use are other criteria of value in assessing bumper repair compositions. Heretofore available compositions have been lacking some or all of these qualities to some degree, but the present composition affords them all and is a breakthrough in the art and science of urethane compositions which are adhesive, sandable, rapid curing, tough, and capable of providing predictable reproducibility in use after use.

From US Re 31 389 a non-wicking polyurethane lasting system is known, which has a high viscosity and short gel-time, so that it does not penetrate into hollow tubes. The product is prepared by prereacting an excess of isocyanate component with a polyol, having a molecular weight between 400-6000 in a ratio of 1 - 1, 1:1 and mixing said prepolymer with a polyol including N,N'-tetra-(2-hydroxypropyl)-ethylen-diamine as a catalyst. The resulting mixture, while hardening fast, has only a moderate hardness.

From US 40 45 527 similar compositions are known, which may also be cast into flexible sheets and include up to 10 % of silicate filter and minor amounts of other additives.

US 3.691.117 discloses a polyurethane composition by reacting a prepolymer of higher molecular weight diol and isocyanate with polyol containing chain extenders. The composition is used to protect surfaces especially against electrolysis on conduction substances.

GB 2147 596 discloses a filled, liquid polyol compound, in which the viscosity is increased by incorporating an alkanolamine. The polyol may be reacted with polyisocyanates and a catalyst to form urethanes, containing said fillers. Elastomeric or foamed products are mainly produced.

It is accordingly an object of the invention to use a novel urethane composition having the above named properties so as to be useful in bumper and auto body repair.

In accordance with the invention, a composition is used for bumper repair and other auto body repair applications which is prepared by mixing a first side comprising an isocyanato reagent under urethane polymer forming conditions simultaneously with a second side comprising a premix of an hydroxylated tertiary amine reagent having a first rate of reaction with the isocyanato reagent and a polyol reagent having a second, slower, rate of reaction with the isocyanato reagent, differentially reacting the premix reagents with the isocyanato reagent in phase segregated polymeric urethane forming relation.

In this and like embodiments, there is included also selecting an isocyanato reagent comprising 4,4'-diphenylmethanediisocyanate; cyclizing the 4,4'-diphenylmethane diisocyanate with itself before mixing for urethane polymer forming reaction; dissolving the cyclized 4,4'-diphenylmethane diisocyanate in noncyclized 4,4'-diphenylmethane diisocyanate before mixing under urethane polymer forming conditions; selecting an isocyanato reagent comprising the polyfunctional isocyanate addition reaction product of an aromatic polyfunctional isocyanate compound and a hydrophobic organic polyfunctional active hydrogen compound, e.g. selecting 4,4'-diphenylmethane diisocyanate as the aromatic polyfunctional isocyanate moiety, cyclizing the 4,4'-diphenylmethane diisocyanate and dissolving it in a solution of 4,4'-diphenylmethane diisocyanate in advance of the addition reaction, selecting Isonate 143-L or Mondur CD as the aromatic polyfunctional isocyanate compound, and selecting hydroxyl-, thiol-, or carboxyl- poly-substituted compounds reactive with isocyanate groups as the hydrophobic organic polyfunctional active hydrogen compound; selecting polytetraalkyleneoxide ether polyols, polyoxyalkyleneoxide ether polyols, aliphatic diols, or active-hydrogen substituted oligomers and fatty acid esters reactive with isocyanate groups as the hydrophobic organic polyfunctional active hydrogen compound; selecting active hydrogen substituted silicone, fluorocarbon, fluorochlorocarbon, polytetraalkyleneoxide ether polyols, acrylic, vinyl, butadiene, cis-polyisoprene, polyamide, polyester, vinyl acetate, acrylamide, polyolefin, or Diels-Alder adducts of unsaturated polyester resin oligomers as the hydrophobic organic polyfunctional active hydrogen compound; also selecting polytetramethyleneoxide ether polyol, D.B castor oil, or hydroxylated glyceryltriricinoleate triester reactive with isocyanate as the hydrophobic organic polyfunctional active hydrogen compound; reacting the 4,4'-diphenylmethane diisocyanate and the hydroxylated glyceryltriricinoleate triester or like reagent in an inert vessel under high shear conditions at a temperature of about 80°C. for about one hour under a vacuum in excess of one millimeter of mercury; effecting the reaction to an amine equivalency in the product of below about 380; selecting as the polyol reagent a polyol preferentially forming a noncrystalline urethane polymer with the isocyanato reagent under urethane polymer forming conditions; as the polyol an hydroxyl-, thiol-, or carboxyl- poly-substituted oligomer having a molecular weight above about 500 and segregated phase defining reaction with the iscyanato reagent relative to said amine reaction under the same urethane polymer forming conditions; selecting a polyoxyalkylene ether polyol as the polyol having a molecular weight above about 1000; reacting the polyol with an isocyanato reagent comprising an adduct of liquid 4,4'-diphenylmethanediisocyanate and glyceryltriricinoleate triester for form a noncrystalline urethane polymer; reacting the polyol and isocyanato reagent adduct in admixture with a tertiary amine having a faster rate of reaction with the isocyanato reagent adduct than does the polyol; selecting as the hydroxylated tertiary amine reagent an alkaryl amine, arylamine, mercaptan, alkylene oxide adduct of alkanol amines, alkoxylated or epoxylated ethylenediamines, triazines, amines and hydrazines having hydroxyl, thiol, or carboxyl functionality; selecting as the hydroxylated tertiary amine reagent a compound having the formula:

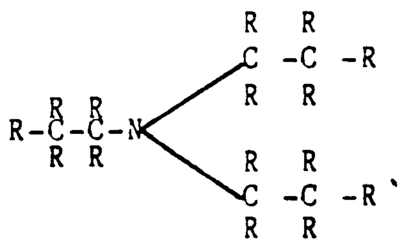

in which at least one R = R1, and each remaining R is R1 or R2 and:
in which:

R1 = -OH; -SH; -N(CH2CH2)OH2; -N(CH2CH3CH2OH)2; -N(CH2CHCH3OH)2.

R2 = -H; -Me; -Alkyl; -OAlk; -OMe; Halogen, -Aryl; -Aroyl

selecting as the hydroxylated tertiary amine reagent the compound N'N'N'N'-tetrakis(2-hydroxypropyl) ethylenediamine; selecting as the isocyanato reagent 4,4'-diphenylmethane diisocyanate, and as the polyol reagent polyoxypropylene polyol triol; reacting the isocyanato reagent with the hydroxylated tertiary amine reagent to a crystalline urethane polymer, and with the polyol reagent to an amorphous polymer interdispersed with the crystalline polymer; employing as the first side per 100 parts by weight from 25 to 75 parts of 4,4'-diphenylmethane diisocyanate, from 3 to 25 parts of hydroxylated tertiary amine, glycerylricinoleate triester or polytetramethyleneoxide ether polyol adducted with the 4,4'-diphenylmethane diisocyanate, and any balance a colorant such as carbon black, or a filler or in amounts up to 5 parts by weight a sanding aid such as silica, and an effective amount of an organic thixotrope; and employing as the second side per 100 parts by weight from from 5 to 40 parts of the polyol, from 5 to 40 parts of the hydroxylated tertiary amine, and the balance zeolite, silica, including silane treated silica, vitreous particulate or mixtures thereof, and a thixatrope.

The invention further contemplates the use of compositions comprising a urethane polymer reaction product of a first side comprising an isocyanato reagent simultaneously with a second side comprising a premix of an hydroxylated tertiary amine reagent and a polyol reagent which are differentially reactive with the isocyanato reagent to form a phase segregated urethane polymer.

In this and like embodiments, isocyanato reagent typically comprises 4,4'-diphenylmethanediisocyanate, the isocyanato reagent comprises cyclized 4,4'-diphenylmethane diisocyanate; the isocyanato reagent comprises the cyclized 4,4'-diphenylmethane diisocyanate dissolved in noncyclized 4,4'-diphenylmethane diisocyanate; the isocyanato reagent comprises the polyfunctional isocyanate addition reaction product of an aromatic polyfunctional isocyanate compound and a hydrophobic organic polyfunctional active hydrogen compound; the aromatic polyfunctional isocyanate compound comprises 4,4'-diphenylmethane diisocyanate; the 4,4'-diphenylmethane diisocyanate is cyclized and dissolved in a solution of 4,4'-diphenylmethane diisocyanate; the compound is Isonate 143-L or Mondur CD; the hydrophobic organic polyfunctional active

hydrogen compound comprises hydroxyl-, thiol-, or carboxyl- poly-substituted compounds reactive with isocyanate groups; the hydrophcbic organic polyfunctional active hydrogen compound comprises polyoxyalkyleneoxide ether polyols, aliphatic diols, or active-hydrogen substituted oligomers and fatty acid esters reactive with isocyanate groups; the hydrophobic organic polyfunctional active hydrogen compound comprises active hydrogen substituted oligomers selected from silicone, fluorocarbon, fluorochlorocarbon, acrylic, vinyl, butadiene, cis-polyisoprene, polyamide, polyester, vinyl acetate, acrylamide, polyolefin, or Diels-Alder adducts of unsaturated polyester resin oligomers; the hydrophobic organic polyfunctional active hydrogen compound comprises hydroxylated glyceryltriricinoleate triester reactive with isocyanate; the 4,4'-diphenylmethane diisocyanate and the hydroxylated glyceryltriricinoleate triester compounds are prereacted in a chemically inert vessel under high shear conditions at a temperature of about 80°C. for about one hour under a vacuum in excess of one millimeter of mercury; the prereacted compounds have an amine equivalency in the product of below about 380; the polyol reagent is a polyol preferentially forming a noncrystalline urethane polymer with the isocyanato reagent under urethane polymer forming conditions; the polyol is an hydroxyl-, thiol-, or carboxyl-poly-substituted oligomer having a molecular weight above about 500 and a segregated phase defining reaction with the iscyanato reagent than the amine reaction with the isocyanato reagent under the same urethane polymer forming conditions; the polyol reagent is a polytetraalkyleneoxide ether polyol or polyoxyalkylene ether polyol; the polyol has a molecular weight above about 1000; the urethane polymer is obtained by reaction of the polyol with an isocyanato reagent comprising an adduct of liquid 4,4'-diphenylmethanediisocyanate and polytetramethyleneoxide ether polyol, D.B. castor oil, or glyceryltriricinoleate triester and is a noncrystalline urethane polymer; tertiary amine reagent has a faster rate of reaction with the isocyanato reagent adduct than does the polyol reagent, whereby the urethane polymer comprises a crystalline portion produced by reaction of the amine and the adduct and a noncrystalline portion being dispersed through the noncrystalline portion; and the hydroxylated tertiary amine reagent comprises an alkylene oxide adduct of alkanol amines, alkoxylated or epoxylated ethylenediamines, trizines, amines and hydrazines having hydroxyl, thiol, or carboyl functionality, preferentially forming a crystalline urethane polymer with the isocyanato reagent under urethane polymer forming conditions; the polyol reagent is a polyol preferentially forming a noncrystalline urethane polymer with the isocyanato reagent under urethane polymer forming conditions, and the hydroxylated tertiary amine reagent a compound has the formula:

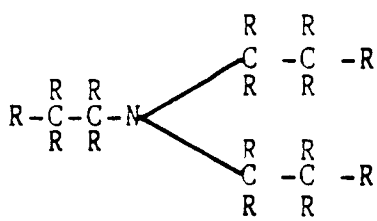

in which at least one R = R1 and each remaining R is R1 or R2, and:

in which:

R1 = -OH; -SH; -N(CH2CH2)OH2; -N(CH2CH3CH2OH)2; -N(CH2CHCH3OH)2.

R2 = -H; -Me; - Alkyl; OAlk; -OMe; - Halogen -Aryl; -Aroyl;

or the polyol reagent is a polyol preferentially forming a noncrystalline urethane polymer with said isocyanato reagent under urethane polymer forming conditions, and the hydroxylated tertiary amine reagent is N'N'N'N'-tetrakis(2-hydroxypropyl) ethylenediamine; the isocyanato reagent is 4,4'-diphenylmethane diisocyanate, and said polyol reagent is polyoxypropylene polyol triol; the urethane polymer phase obtained by reaction of the isocyanato reagent with the hydroxylated tertiary amine reagent is a crystalline urethane polymer, and the urethane polymer obtained by reaction of said isocyanato reagent with said polyol reagent is an amorphous polymer phase interdispersed with said crystalline polymer; the polymer comprises per 200 parts by weight from 20 to 75 parts of 4,4'-diphenylmethane diisocyanate, e.g. Mondur CD, from 10 to 50 parts of polytetramethyleneoxide ether polyol, D.B. castor oil, or glycerylricinoleate triester adducted with said 4,4'-diphenylmethane diisocyanate, from from 0 to 30 parts of said polyol, from 10 to 60 parts of said hydroxylated tertiary amine, and the balance paste adjuvants comprising silicas, zeolite, glass, organic thixotropes, and the like in effective amounts for the intended use.

In the use of the foregoing compositions in auto body repair, they are formed in situ against a rigid substrate and reacting to the urethane polymer composition comprising interdispersed crystalline and

noncrystalline portions of the polymer.

Preferred Modes

The term auto body component herein refers to bumpers, fenders, trunk lids, hoods and other auto body parts which are used in replacement of sheet metal or otherwise, and which are subject to gouging, cracking, splitting, and like surface damage. Typically such parts are formed of urethane polymers, but various other polymeric compositions may also be used. The present repair composition is widely adherent when cured and can be used to bond itself or another material to a first material, and can be usefully formed in situ or preformed and then adhered to a substrate. The manifold uses of the present compositions are limited only by the needs in the marketplace for a tough urethane polymer capable of quick curing to a finishable state and reliably adhering to a chosen substrate.

As noted above, urethanes for bumper repair have been less hard than presently desirable. The use of higher concentrations of isocyanate reagent on the A side will increase hardness, but only at the cost of slower cure. It has now been discovered however that the use of a two-stage B side, one part of which is a tertiary amine as hereinafter described, enables harder product and with the rapid cure times to sandability needed.

In particular, the invention systems use a modified isocyanate reagent and in certain formulations intended for auto component repair maintains a 2.3 or less isocyanate functionality per mole, e.g. a 1.8 to 2.3 functionality on diphenylmethane diisocyanate, less than that realized with polymeric isocyanates. Functionality herein refers the number of equivalents of isocyanate per mole. Preferred as the isocyanate reagent is a cyclized diphenylmethane diisocyanate dissolved in noncyclized diphenylmethane diisocyanate such as is commercially available as Mondur CD.

It has been found that whereas a prepolymer of this diisocyanate having an amine equivalence of about 400 when reacted with the B side at a 1:1 volume ratio forms a rubbery, soft urethane, e.g. having a Shore A hardness of 65-85. Reducing the amine equivalence to 380 or less and reacting with an equivalent volume amount of the B side, results in a reaction which is initially quite fast giving a faster work time. In the invention compositions there is a balance between work time and sanding readiness while obtaining a urethane final hardness similar to the substrate. Critical to this is the use of the tertiary amine described herein on the A side, which enables sanding without galling or reversion of the polymer with sanding heat.

Illustrative isocyanate compounds herein are: toluene diisocyanate (2,4-and 2-6 isomers and blends thereof), naphthylene diisocyanate, isophorone diisocyanate, methylenedicyclohexyldiisocyanate, hexamethylene diisocyanate, m-xylidenediisocyanate, paraphenylene diisocyanate, trimethylxylidene diisocyanate, cyclohexane diisocyanate, ortho-, meta- and para-phenylenediisocyanate, trimethylxylidenediisocyanate, 3,3' dimethyl 4,4'-diphenylmethane diisocyanate, 3,3'- dimethyl- 4,4'-biphenyldiisocyanate , and 3/3'- dimethoxy - 4,4'-diisocyanate, dianisidine diisocyanate.

Illustrative amines herein are: alk-aryl amines; methylenediphenylmethanediamine, methylene-bis-2-chloroaniline, 4,4' -methylenediphenylsulphone-diamine, 4,4'-methylanthranilate, 2-bis (2'-aminophenylthio)-ethane, triethyleneglycol-bis (4-amino benzoate), melamine, ethyloxylated aniline, propoxylated aniline,

Illustrative polyols include ethyleneglycol, butanediol, trimethylol ethane, trimethylol propane, dipropylene glycol, propylene glycol, ethylene glycol, butenediol, butynediol, trimethylol propane, cyclohexanedimethylol, 1,4-di-hydroxycyclobutane, pentaerythritottetramercaptoproprionate, hexanediol, and b-dihydroxyethylether-hydroquinone.

The present compositions have typically hardnesses of 80 Shore A to 80 Shore D and are useful for the repair of urethane, SMC ABS, vinyl, fiberglass, epoxy, styrene, vulcanized natural neoprene, nitrile, SBR, EPR, EVA and EPDM rubber, metal, glass, cement, wood, ceramic, for auto body and rubber bumper repair, and general repair. The compositions have fast curability so that sanding can be accomplished within 15 minutes, even on thin edges without melting of the urethane, and exhibit the inherent resistance of urethanes to delamination because of their tenaceous adhesion to metal, plastic, rubber, fiberglass, and urethane substrates and virtually all substrates which have any active hydrogen functionality- which is virtually all surfaces except polyolefins and Teflon-type surfaces. Moreover, they resist delamination because the urethanes do not go through their glass transition during normal service temperature as epoxies do or degrade in humid and acid conditions as epoxy repair materials do.

In preparation, the first step is the synthesis of the Part A and Part B components. For the Part A component: 4,4'-diphenylmethane diisocyanate (sometimes MDI) is converted into a cyclized form with itself. This cyclized structure is then dissolved in MDI to produce a storage-stable liquid form called liquid MDI having an overall isocyanate functionality of 2.1 to 2.2. Pure 4,4'-diphenylmethane diisocyanate could have been selected to produce the prepolymer, but this special form was used as the reactive isocyanate in

order to produce a more storage-stable solution (stable towards freezing during storage). This liquid MDI form is commercially avaiable from Upjohn Company as Isonate 143-L or from Mobay Chemical as Mondur CD.

A quasi prepolymer is synthesized from the addition reaction of liquid MDI (Mondur CD) and preferably polytetramethyleneoxide ether polyol, D.B. castor oil, or gylceryltriricinoleate triester (sometimes GTR). Placed into the reaction solution was fumed silica of a 0.04 microm size. This composition was placed in an inert reaction vessel which was capable of heating the reaction mixture, controlling its reaction temperature, high-shear mixing, and a vacuum exceeding one millimeter. The ingredients were high-shear mixed and heated to 80-85 degrees centigrade for one hour. During this time, a vacuum was pulled on the mixture in excess of one millimeter of mercury. The amine equivalent was measured and the synthesized mixture packaged in metal squeeze tube containers.

The Part A (prepolymer) component utilizes the extremely hydrophobic glyceryltriricinoleate triester hydroxyl-functional compound, e.g. a refined castor oil. Other hydroxyl-functional compounds which could have been selected to achieve this hydrophobic property include such compounds as polyoxytetramethyleneoxide ether polyols, polyoxypropylene ether polyols, cyclohexanedimethylol, hexanediol, dipropylene glycol, tripropylene glycol, propylene glycol, ethylene glycol, diethyleneglycol, triethylene glycol, 1,3-butanediol, butanediol, propargyl alcohol, butyne diol, and the family of di- and tri-functional monomers or polyols, as well as silicone-, flurocarbon-, fluorochlorocarbon-, acrylic-, vinyl-, butadiene-, cis-polyisoprene-, polyamide-, polyimide-, Diels-Alder adducts of unsaturated polyester resin-, polyester resins, vinyl acetate-, acrylamide-, polyolefin-, and any combination of the above oligomers modified to have active-hydrogen functionality. Carboxylic acid-functional-, thiol functional- and other active-hydrogen- functional oligomers or monomers can also be selected to be reacted with the isocyanate to form the prepolymer used as the isocyanato reagent.

The Part B (polyol reagent) is preferably partially composed of a high molecular weight polyol oligomer, e.g a 500 to 1000 up to 6000 molecular weight and higher liquid tri-functional polyoxypropylene ether polyol having some ethylene oxide capping to give secondary functionality. Any modification of the foregoing hydrophobic compounds may be selected as long as the reactivity of the polyol component its reactivity is slower than the tertiary amine coreactant or such as to define a phase segregated polymer relative to that defined by the amine reaction with isocyanate during formation of the polymer, so that the polyol forms essentially (i.e. thermodynamically) random structure by virtue having little ability to crystallize, or organize its structure, and it has the correct solubility to phase-segregate from the crystalline amine isocyanate adduct phase and to thereby form multi-phase matrix structures. It addition the polyol should have sufficient functionality to crosslink with the crystalline "zones" even if the user should mix the Part A and Part B components off-ratio enough to cause the cross-link density to be reduced, should have some tendency to cyclize or helicize, or form polymeric strands which are capable of being elongated when the multi-phase structure is stressed by an outside force, and most importantly, have the ability to return to its random or amorphous structure once the external force is relieved.

In combination with the just-described polyol is a hydroxyl-reactive amine compound capable of forming highly crystallized and ordered structures upon reaction with the isocyanate functionality in the Part A component. The amine reagent preferably is a somewhat ordered structure containing tertiary amine groups. While not wishing to be bound to any particular theory of operation, it is theorized that the tertiary amino groups of amine reagent herein, having a free-electron pair, orients that electron pair with some other moiety in the polymer solution (in its prepolymerized form) to resist unwanted melting during sanding in small, less than one gram aliquots, whereby structures of adducts can be visualized which show a high crystalline and oriented structure capable of withstanding many kilocalories of input heat during a sanding process, and the highly organized nature of these crystalline zones can be supposed to have sufficient intramolecular forces to remain intact, while only amorphous zones would be unsupportive at their interstices and consquently "ablate" during the sanding process. In general it is significant that only the tertiary amino groups, not having reactive hydrogen functionality on the amino groups themselves in order to withstand instant reactivity, function herein as the amine reagent. In addition, the tertiary amino groups must have hydroxyl functionality adducted. The best means of adducting is to use ethylene oxide or propylene oxide so that only one ethylene or one propylene is adducted to each active hydrogen of the tertiary amino group. Illustrative amine reagents herein are: triethanol amine; tripropanol amine; combinations of diethanol-monopropanol amine, etc.; ethylenediamine tetra ethyoxylate; ethylenediamine tetrapropoxylate; ethoxylated and propoxylated 1,3,5-triazines, or other triazine isomers; cyclic combinations of ethylenediamine, hydrazine, amines which are ethoxylated, propoxylated or epoxidized in any form which leaves hydroxyl, or thiol functionality.

The Part B side also contains a zeolite, such as a sodiumaluminosilicate zeolite structure, e.g. capable

of containing at least one molecule of water within its clathrate structure. It has been found that levels of zeolite substantially exceeding 5 percent up to as much as 85% substantially improve the working properties of the invention composition, and substantially improve the physical properties of the composition for finishing. Again, it is theorized that the zeolite is acting synergistically with the amine reagent in producing the required "through-cure" and "ablative" properities so significantly present in the invention composition.

The composition ingredients are high-shear mixed in the vessel, heated to 105-110 degress Centigrade in order to ensure that all water is removed from the mixture. The filled polyol component was packaged in its own separate metal squeeze tube container for storage.

The composition of the Part A and Part B pastes when extruded and mixed desirably produce the correct viscosity pastes for extruding out of a number 10 orifice from a metal squeeze tube at nearly equal and controlled diameters. By extruding equal length lines of pastes the volume ratios are maintained at roughly 1.00 to 1.00. The achievement of control of the mix ratios is very important for maintenance of the stoichiometry of the reactive components and for achieving maximum molecular weight polymers. The composition is preferably built upon, e.g. the trifunctional ricinoleate, trifunctional high-molecular weight oligomer, and the tetra-functional N,N,N,N-tetrakis(2-hydroxyethyl or propyl)ethylenediamine in order to achieve an extremely high level of off-ratio or poor mixing forgiveness.

The Part A component, composed of 4,4'-diphenylmethane diisocyanate and 4,4'-diphenylmethane diisocyanateglyceryltriricinoleate triester prepolymer, reacts first with the active hydrogen groups (hydroxyls) on the tertiary amine hard segment crosslinkers of the Part B component. The reaction of 4,4'-diphenylmethane diisocyanate is fast with the tertiary amine in comparison with the reactivities of the 4,4'-diphenylmethane diisocyanate prepolymers and the polyol reagent compounds. The fast reaction produces crystalline hard segments which align into morphological phases within the unreacted or partially-reacted polyol and 4,4'-diphenylmethane diisocyanate prepolymer phases. This crystalline composition within the liquid amorphous phases produces the malleable consistency of the mixture which occurs immediately after the start of mixing. Condensing the polymer at this point does not fracture the interstices because the amorphous phases have not yet cross linked with the crystalline phases. The forces of condensation merely cause laminar flow and alignment of the hard segments in the liquid soft segment medium. Polymerization continues until the soft segments crosslink the various hard segments and the polymer becomes intractable.

The fully-cured composition is a multi-phase matrix where the crystalline zones (phases) of the matrix are contained within the amorphous zones. The crystalline zones are tied to the amorphous zones through the prepolymer portion of the Part A component. The multi-phase matrix has the capability of uptaking external forces through thermodynamic ordering of the amorphous zones. The polyol has the capability of uptaking these stresses because the pendant methyl groups on e.g. the polyoxypropylene ether polyol provide barriers to rotation which can be easily overcome by the forces of deformation to produce a B-pleated sheet conformer if the need for uptaking work energy in the form of ordering (negative entropy) is required. Moreover, the pendant methyl groups provide only a low resistance to barriers of rotation allowing the number of possible structures to be high (high randomness) when external forces are relieved.

The composition continues to harden at a controlled rate until nearly full properties are achieved after 2 hours. Full properties are achieved after 24 hours.

<u>Example</u>

<u>Example 1</u>

Part A:

| Material | Eq. Wt. | Equiv. | Wt. % |
|---|---|---|---|
| Mondur CD | - | .430 | 62.0 |
| Polyether polyol | - | .007 | 15.0 |
| D.B. Castor Oil | - | .057 | 18.0 |
| Carbon black | - | - | 1.0 |
| Fumed silica | - | - | 3.0 |
| Organic Thixatrope | - | .003 | 1.0 |
| | 274 | .363 | 100 |

PART B:

| Material | Eq. Wt. | Equiv. | Wt. % |
|---|---|---|---|
| Polyether Polyol | - | .015 | 30.0 |
| N,N,N,N-tetrakis(2-hydroxypropyl) ethylenediamine | - | .342 | 25.0 |
| Sodium Aluminuncsilicate Zeolite Powder | - | - | 8.0 |
| Quartz silica | - | - | 34.0 |
| Organic Thixatrope | - | - | 3.0 |
| | 280 | .363 | 100 |

The composition was mixed in equal volume amounts and placed against a gouge in a bumper, increased in hardness until in minutes it was easily carvable and shapeable. After 15 minutes, it was at the hardness to be sandable without harm to the filled in composition. The hardness properties continued to build gradually to form a very hard elastomer after one hour when it reached nearly full properties. Full hardness properties were reached after 24 hours.

**Claims**

1. The use of a composition for auto component repair, comprising two components (A) and (B) which form a phase segregated urethane polymer reaction product in which

   (A) is a premix of a polyfunctional isocyanate prepolymer prepared from an aromatic polyfunctional isocyanate compound and a hydrophobic organic polyfunctional active hydrogen compound and non

reacted diisocyanate, this premix having an amine equivalent weight of 380 or less and
(B) a premix of a hydroxylated tertiary amine reagent and a polyol reagent, in which the reactivity of the polyol towards (A) is slower than the reactivity of the amine.

**2.** The use of a composition according to claim 1, including in component (B) per 100 parts by weight from 5 to 40 parts of the polyol, 5 to 40 parts of the hydroxylated tertiary amine and the balance a sanding aid as zeolite, silica, vitreous particulates and/or organic thixotropes.

**3.** The use of a composition according to claim 2, in which said aromatic polyfunctional isocyanate compound comprises 4,4'-diphenylmethane diisocyanate.

**4.** The use of a composition according to claim 3, in which said 4,4'-diphenylmethane diisocyanate is cyclized with itself and dissolved in noncyclized 4,4'-diphenylmethane diisocyanate.

**5.** The use of a composition according to claim 2, in which said hydrophobic organic polyfunctional active hydrogen compound comprises hydroxyl-, thiol-, or carboxyl- poly-substituted compounds reactive with isocyanate groups.

**6.** The use of a composition according to claim 5, in which said hydrophobic organic polyfunctional active hydrogen compound comprises polytetraalkyleneoxide ether polyols or polyoxyalkyleneoxide ether polyols, aliphatic diols, or active-hydrogen substituted oligomers and fatty acid esters reactive with isocyanate groups.

**7.** The use of a composition according to claim 6, in which said hydrophobic organic polyfunctional active hydrogen compound comprises active hydrogen substituted oligomers selected from silicone, fluorocarbon, fluorochlorocarbon, polyether polyols, polytetraalkyleneoxide ether polyols, methacrylic, vinyl, butadiene, cis-polyisoprene, polyamide, polyester, vinyl acetate, acrylamide, polyolefin, or Diels-Alder adducts of unsaturated polyester resin oligomers.

**8.** The use of a composition according to claim 2, in which said hydrophobic organic polyfunctional active hydrogen compound comprises polytetramethyleneoxide ether polyols, D.B. castor oil, hydroxylated glyceryltriricinoleate triester reagent reactive with isocyanate.

**9.** The use of a composition according to claim 1, in which said polyol has a molecular weight above about 1000.

**10.** The use of a composition according to claim 9, in which the urethane polymer is obtained by reaction of said polyol with an isocyanato reagent comprising an adduct of liquid 4,4'-diphenylmethanediisocyanate and polytetramethyleneoxide ether polyol, D.B. castor oil, or glyceryltriricinoleate triester and is a noncrystalline urethane polymer.

**11.** The use of a composition according to claim 9, in which tertiary amine reagent has a faster rate of reaction with said isocyanato reagent adduct than does said polyol reagent, whereby said urethane polymer comprises a crystalline phase produced by reaction of said amine and said adduct and a noncrystalline phase produced by reaction of said polyol and said adduct, said crystalline portion being dispersed through said noncrystalline portion.

**12.** The use of a composition according to claim 1, in which said polyol reagent is a polyol preferentially forming a noncrystalline urethane polymer phase with said isocyanato reagent under urethane polymer forming conditions, and said hydroxylated tertiary amine reagent comprises an alkaryl amine, arylamine, mercaptan or alkylene oxide adduct of alkanol amines, alkoxylated or epoxylated ethylenediamines, triazines, amines and hydrazines having hydroxyl, thiol, or carboxyl functionality preferentially forming a crystalline urethane polymer with said isocyanato reagent under urethane polymer forming conditions.

**13.** The use of a composition according to claim 1, in which said polyol reagent is a polyol preferentially forming a non-crystalline urethane polymer with said isocyanato reagent under urethane polymer forming conditions, and said hydroxylated tertiary amine reagent a compound has the formula:

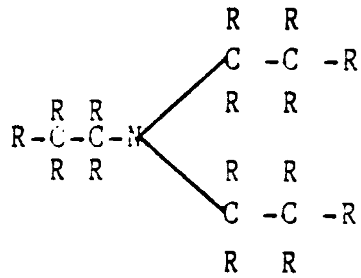

in which at least one R = R1, and each remaining R is R1 or R2, and:
in which
R1 = -OH; -SH; -N(CH2CH2)OH2; -N(CH2CH3CH2OH)2; -N(CH2CHCH3OH)2.
R2 = -H; -Me; - Alkyl; OAlk; -OMe; -Halogen, -Aryl; -Aroyl;

**14.** The use of a composition according to claim 1, in which said polyol reagent is a polyol preferentially forming a non-crystalline urethane polymer with said isocyanato reagent under urethane polymer forming conditions, and the hydroxylated tertiary amine reagent is N'N'N'N'-tetrakis (2-hydroxypropyl) ethylenediamine.

**15.** The use of a composition according to claim 14, in which said isocyanato reagent is 4,4'diphenylmethane diisocyanate, and said polyol reagent is polyoxypropylene polyol triol.

**16.** The use of a composition according to claim 1, in which the urethane polymer phase obtained by reaction of said isocyanato reagent with said hydroxylated tertiary amine reagent is a crystalline urethane polymer, and the urethane polymer obtained by reaction of said isocyanato reagent with said polyol reagent is an amorphous polymer phase interdispersed with said crystalline polymer.

**17.** The use of a composition according to claim 1, in which said polymer comprises per 200 parts by weight from 15 to 45 parts of 4,4'-diphenylmethane diisocyanate, from 3 to 8 parts of polytetramethyleneoxide ether polyol, D.B. castor oil, or glycerylricinoleate triester adducted with said 4,4'-diphenylmethane diisocyanate, from from 0 to 30 parts of said polyol, from 10 to 60 parts of said hydroxylated tertiary amine, and the balance a hardening filler.

## Patentansprüche

**1.** Die Verwendung einer Mischung zum Reparieren von Automobilteilen, welche zwei Komponenten (A) und (B) umfaßt, welche ein phasenabgesondertes Urethanpolymerisationsprodukt bilden, in welchem
(A) eine Vormischung eines polyfunktionalen Isocyanatprepolymers ist, welches aus einem aromatischen, polyfunktionalen Isocyanat und einer hydrophoben, organischen, polyfunktionalen, aktiven Wasserstoff enthaltenden Verbindung und nichtreagiertem Diisocyanat hergestellt ist, wobei dieses Premix ein Aminäquivalentgewicht von 380 oder weniger besitzt und
(B) ein Premix eines hydroxylierten tertiären Aminreagenzes und eines Polyolreagenzes ist, in welchem die Reaktivität des Polyols gegenüber (A) langsamer ist, als die Reaktivität des Amins.

**2.** Die Verwendung einer Mischung gemäß Anspruch 1, welche in der Komponente (B) auf 100 Gewichtsteile von 5 bis 40 Teile des Polyols, 5 bis 40 Teile des hydroxylierten tertiären Amins und als Rest Schleifhilfen, wie Zeolit, Quarz, Glasteilchen und/oder organische Tixotropiemittel, enthält.

**3.** Die Verwendung einer Mischung gemäß Anspruch 2, in der die aromatischen, polyfunktionalen Isocyanatverbindungen 4,4'-Diphenylmethandiisocyanatdarstellen.

**4.** Die Verwendung einer Mischung gemäß Anspruch 3, in welcher das 4,4'-Diphenylmethandiisocyanat mit sich selbst cyclisiert und in nichtcyclisiertem 4,4'-Diphenylmethandiisocyanat gelöst ist.

**5.** Die Verwendung einer Mischung gemäß Anspruch 2, in welcher die hydrophobe, organische, polyfunktionale, aktiven Wasserstoff enthaltende Verbindung, hydroxyl-, thiol-, oder carboxyl-, polysubstituierte

Verbindungen umfaßt, welche mit Isocyanatgruppen reagieren.

6. Die Verwendung einer Mischung gemäß Anspruch 5, in welcher die hydophoben, organischen, polyfunktionalen, aktiven Wasserstoff enthaltenden Verbindungen Polytetraalkylenoxidetherpolyole oder Polyoxyalkylenoxidetherpolyole, aliphatische Diole oder mit aktivem Wasserstoff substituierte Oligomere und Fettsäureester, welche mit Isocyanatgruppen reagieren, umfaßt.

7. Die Verwendung einer Mischung gemäß Anspruch 6, in welcher die hydrophobe, organische, polyfunktionale, aktiven Wasserstoff enthaltende Verbindung durch aktiven Wassertoff substituierte Oligomere ausgewählt aus Silikonen, Fluorkohlenstoffen, Fluorchlorkohlenstoffen, Polyetherpolyolen, Polytetraalkylenetherpolyolen, Metacryl, Vinyl, Butadien, cis-Polyisopren, Polyamid, Polyester, Vinylacetat, Acrylamid, Polyolefin oder Diels-Alder Addukte von ungesättigten Polyesterharz-Oligomeren umfaßt.

8. Die Verwendung einer Mischung gemäß Anspruch 2, in welcher die hydrophobe, organische, polyfunktionale, aktiven Wasserstoff enthaltende Verbindung Polytetramethylenoxidetherpolyole, Ricinusöl, hydroxilierte Glyceryltriricinoleattriester, welche mit Isocyanaten reagieren, umfaßt.

9. Die Verwendung einer Mischung gemäß Anspruch 1, in welcher das Polyol ein Molekulargewicht von ungefähr 1000 hat.

10. Die Verwendung einer Mischung gemäß Anspruch 9, in welcher das Urethanpolymer erhalten wird durch Reaktion des Polyols mit einem Isocyanatreagenz, welches ein Addukt von flüssigem 4,4'-Diphenylmethandiisocyanat und Polytetramethylenoxidetherpolyol, Ricinusöl, oder Glyceryltriricinoleattriester umfaßt und ein nichtkristallines Urethanpolymer darstellt.

11. Die Verwendung einer Mischung gemäß Anspruch 9, in welcher das tertiäre Aminreagenz eine höhere Reaktionsrate mit dem Isocyanatreagenzaddukt hat, als das Polyolreagenz, wobei das Urethanpolymer eine kristalline Phase umfaßt, die durch Reaktion des Amins und des Adduktes hergestellt ist und eine nichtkristalline Phase, die durch Reaktion des Polyols mit dem Addukt hergestellt ist, wobei der kristalline Anteil in dem nichtkristallinen Anteil dispergiert ist.

12. Die Verwendung einer Mischung gemäß Anspruch 1, in welcher das Polyolreagenz ein Polyol ist, welches vorzugsweise eine nichtkristalline Urethanpolymerphase mit dem Isocyanat unter Urethanpolymer-bildenden Bedingungen bildet und das hydroxilierte tertiäre Aminreagenz ein Alkarylamin, Arylamin, Mercaptan oder Alkylenoxidaddukt von Alkanolaminen, alkoxylierten oder epoxylierten Ethylendiaminen, Triazinen, Aminen und Hydrazinen mit Hydroxyl-, Thiol-, oder Carboxylfunktionen darstellt, welches vorzugsweise ein kristallines Urethanpolymer mit dem Isocyanatreagenz unter Urethanpolymer-bildenden Bedingungen bildet.

13. Die Verwendung einer Mischung gemäß Anspruch 1, in welcher das Polyolreagenz ein Polyol ist, welches vorzugsweise ein nichtkristallines Urethanpolymer mit dem Isocyanatreagenz unter Urethanpolymer-bildenden Bedingungen bildet, wobei das hydroxilierte,tertiäre Aminreagenz eine Verbindung der Formel

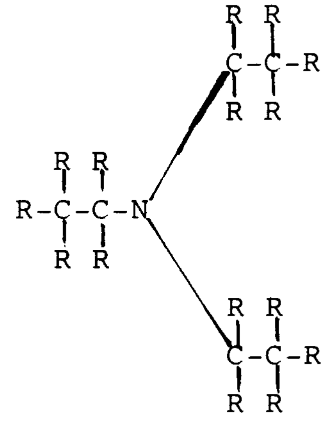

in welcher mindestens ein R = R1 ist,
und die verbleibenden R = R1 oder R2 sind und
in welcher

R1 = -OH, -SH, $-N(CH_2CH_2OH)_2$, $-N(CH_2CH_3CH_2OH)_2$, $-N(CH_2CHCH_3OH)_2$
R2 = -H, -Me, -Alkyl, -OAlkyl, -OMe, -Halogen, -Aryl, -Aroyl

bedeutet.

**14.** Die Verwendung einer Mischung gemäß Anspruch 1, in welcher das Polyolreagenz ein Polyol ist, welches vorzugsweise ein nichtkristallines Urethanpolymer mit dem Isocyanatreagenz unter Urethanpolymer-bildenden Bedingungen bildet und das hydroxylierte,tertiäre Aminreagenz N'N'N'N'-Tetrakis-(2-hydroxypropyl)ethylendiamin ist.

**15.** Die Verwendung einer Mischung gemäß Anspruch 14, in welcher das Isocyanatreagenz 4,4'-Diphenylmethandiisocyanat und das Polyolreagenz Polyoxypropylenpolyoltriol ist.

**16.** Die Verwendung einer Mischung gemäß Anspruch 1, in welcher die Urethanpolymerphase, die durch Reaktion des Isocyanatreagenzes mit dem hydroxylierten tertiären Aminreagenz erhalten wird, ein kristallines Urethanpolymer ist und das Urethanpolymer, daß durch Reaktion des Isocyanatreagenzes mit dem Polyolreagenz erhalten wird, eine amorphe Polymerphase ist, welche mit dem kristallinen Polymer vermischt ist.

**17.** Die Verwendung einer Mischung gemäß Anspruch 1, in welcher das Polymer auf 200 Gewichtsteile von 15-45 Teilen 4,4'-Diphenylmethandiisocyanat, von 3-8 Teilen Polytetramethylenoxidetherpolyol, Ricinusöl oder Glycerylricinoleattriesteraddukt mit dem 4,4'-Diphenylmethandiisocyanat und von 0-30 Teile des Polyols, von 10-60 Teile des hydroxylierten tertiären Amins und den Rest einen härtenden Füllstoff enthält.

## Revendications

**1.** Utilisation d'une composition pour réparation de pièces d'automobile, comprenant deux composants (A) et (B) qui forment un produit réactionnel de type polyuréthanne à phases séparées, dans laquelle
(A) est un prémélange d'un prépolymère à fonctionnalité isocyanate polyfonctionnel préparé à partir d'un isocyanate aromatique polyfonctionnel et d'un composé organique hydrophobe à hydrogène actif polyfonctionnel, et de diisocyanate n'ayant pas réagi, ce prémélange ayant un poids équivalent d'amine de 380 ou moins et
(B) est un prémélange d'un corps réagissant du type amine tertiaire hydroxylée et d'un corps réagissant du type polyol, dans lequel la réactivité du polyol vis-à-vis de (A) est plus lente que la réactivité de l'amine.

**2.** Utilisation d'une composition selon la revendication 1, dont le composant (B) comprend, pour 100 parties en poids, 5 à 40 parties du polyol, 5 à 40 parties de l'amine tertiaire hydroxylée et le reste d'un adjuvant de ponçage tel qu'une zéolite, de la silice, des particules vitreuses et/ou des agents thixotropes organiques.

**3.** Utilisation d'une composition selon la revendication 2, dans laquelle ledit isocyanate aromatique polyfonctionnel comprend du 4,4'-diisocyanato-diphénylméthane.

**4.** Utilisation d'une composition selon la revendication 3, dans laquelle ledit 4,4'-diisocyanato-diméthylméthane est cyclisé avec lui-même et dissous dans du 4,4'-diisocyanato-diphénylméthane non cyclisé.

**5.** Utilisation d'une composition selon la revendication 2, dans laquelle ledit composé organique hydrophobe à hydrogène actif polyfonctionnel comprend des composés polysubstitués par des groupes hydroxyle, thiol ou carboxyle, réactifs avec les groupes isocyanato.

**6.** Utilisation d'une composition selon la revendication 5, dans laquelle ledit composé organique hydrophobe à hydrogène actif polyfonctionnel comprend des poly(oxyde de tétraalkylène)-polyols ou des poly-(oxyde d'alkylène)-polyols, des diols aliphatiques ou des oligomères et esters d'acides gras substitués par de l'hydrogène actif réactifs avec les groupes isocyanato.

7. Utilisation d'une composition selon la revendication 6, dans laquelle ledit composé organique hydrophobe à hydrogène actif polyfonctionnel comprend des oligomères substitués par de l'hydrogène actif choisis parmi les silicones, fluorocarbures, fluorochlorocarbures, polyétherpolyols, poly(oxyde de tétraalkylène)-polyols, composés méthacryliques, composés vinyliques, butadiène, cis-polyisoprènes, polyamides, polyesters, acétate de vinyle, acrylamide, polyoléfines ou produits d'addition de Diels-Alder d'oligomères de résine polyester insaturée.

8. Utilisation d'une composition selon la revendication 2, dans laquelle ledit composé organique hydrophobe à hydrogène actif polyfonctionnel comprend des poly(oxyde de tétraméthylène)-polyols, l'huile de ricin D.B., du triricinoléate de glycéryle hydroxylé réactif avec l'isocyanate.

9. Utilisation d'une composition selon la revendication 1, dans laquelle ledit polyol a un poids moléculaire supérieur à 1000 environ.

10. Utilisation d'une composition selon la revendication 9, dans laquelle le polyuréthanne est obtenu par réaction dudit polyol avec un corps réagissant du type isocyanate comprenant un produit d'addition de 4,4'-diisocyanato-diphénylméthane liquide et de poly(oxyde de tétraméthylène)-polyol, d'huile de ricin D.B. ou d'un triricinoléate de glycéryle, et c'est un polyuréthanne non cristallin.

11. Utilisation d'une composition selon la revendication 9, dans laquelle le corps réagissant du type amine tertiaire présente une plus grande vitesse de réaction avec ledit produit d'addition réagissant du type isocyanate que ledit corps réagissant du type polyol, si bien que ledit polyuréthanne comprend une phase cristalline produite par réaction de ladite amine et dudit produit d'addition et une phase non cristalline produite par réaction dudit polyol et dudit produit d'addition, ladite portion cristalline étant dispersée dans ladite portion non cristalline.

12. Utilisation d'une composition selon la revendication 1, dans laquelle ledit corps réagissant du type polyol est un polyol formant préférentiellement une phase de polyuréthanne non cristalline avec ledit corps réagissant du type isocyanate dans des conditions de formation de polyuréthanne, et ledit corps réagissant du type amine tertiaire hydroxylée comprend une alkarylamine, une arylamine, un mercaptan ou un produit d'addition d'oxyde d'alkylène sur des alcanolamines, des éthylènediamines alcoxylées ou époxydées, des triazines, des amines et des hydrazines ayant une fonctionnalité hydroxyle, thiol ou carboxyle formant préférentiellement un polyuréthanne cristallin avec ledit corps réagissant du type isocyanate dans des conditions de formation de polyuréthanne.

13. Utilisation d'une composition selon la revendication 1, dans laquelle ledit corps réagissant du type polyol est un polyol formant préférentiellement un polyuréthanne non cristallin avec ledit corps réagissant du type isocyanate dans des conditions de formation de polyuréthanne, et ledit corps réagissant du type amine tertiaire hydroxylée est un composé répondant à la formule :

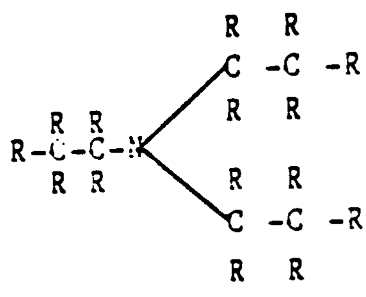

dans laquelle au moins un R = $R^1$, et chaque R restant est $R^1$ ou $R^2$, et :
où
$R^1$ = -OH ; -SH ; $-N(CH_2CH_2)OH_2$ ; $-N(CH_2CH_3CH_2OH)_2$ ; $-N(CH_2CHCH_3OH)_2$,
$R^2$ = -H ; -Me ; -Alkyle ; OAlk ; -OMe ; -Halogène, -Aryle ; -Aroyle.

14. Utilisation d'une composition selon la revendication 1, dans laquelle ledit corps réagissant du type polyol est un polyol formant préférentiellement un polyuréthanne non cristallin avec ledit corps

réagissant du type isocyanate dans des conditions de formation de polyuréthanne, et le corps réagissant du type amine tertiaire hydroxylée est la N',N',N',N'-tétrakis(2-hydroxypropyl)-éthylènediamine.

**15.** Utilisation d'une composition selon la revendication 14, dans laquelle ledit corps réagissant du type isocyanate est le 4,4'-diisocyanato-diphénylméthane, et ledit corps réagissant du type polyol est un polyoxypropylène-triol.

**16.** Utilisation d'une composition selon la revendication 1, dans laquelle la phase de polyuréthanne obtenue par réaction dudit corps réagissant du type isocyanate avec ledit corps réagissant du type amine tertiaire hydroxylée est un polyuréthanne cristallin, et le polyuréthanne obtenu par réaction dudit corps réagissant du type isocyanate avec ledit corps réagissant du type polyol est une phase de polymère amorphe entremêlée avec ledit polymère cristallin.

**17.** Utilisation d'une composition selon la revendication 1, dans laquelle ledit polyuréthanne comprend, pour 200 parties en poids, 15 à 45 parties de 4,4'-diisocyanato-diphénylméthane, 3 à 8 parties de poly-(oxyde de tétraméthylène)-polyol, d'huile de ricin D.B. ou de triricinoléate de glycéryle sous forme de produit d'addition avec ledit 4,4'-diisocyanato-diphénylméthane, 0 à 30 parties dudit polyol, 10 à 60 parties de ladite amine tertiaire hydroxylée, le reste d'une charge de durcissement.